## Europäisches Patentamt

## European Patent Office

## Office européen des brevets

(11) Publication number: **0 147 378**
**B1**

(12) **EUROPEAN PATENT SPECIFICATION**

(45) Date of publication of patent specification: **06.05.87**

(21) Application number: **84870160.3**

(22) Date of filing: **30.11.84**

(51) Int. Cl.⁴: **C 08 K 5/34**, C 07 D 215/00, C 08 G 12/00, C 08 L 21/00

(54) Liquid hydroquinoline-type antioxydants.

(30) Priority: **23.12.83 US 565069**

(43) Date of publication of application:
**03.07.85 Bulletin 85/27**

(45) Publication of the grant of the patent:
**06.05.87 Bulletin 87/19**

(84) Designated Contracting States:
**BE DE FR GB IT**

(56) References cited:
**GB-A-2 002 783**
**US-A-3 413 253**
**US-A-4 158 000**

(73) Proprietor: **MONSANTO COMPANY**
**Patent Department 800 North Lindbergh Boulevard**
**St. Louis, Missouri 63166 (US)**

(72) Inventor: **Evans, David Lane**
**1326 South Milwaukee Street**
**Denver Colorado 80210 (US)**
Inventor: **Merten, Helmut Ludwig**
**1660 Mayflower Lane**
**Hudson Ohio 44236 (US)**

(74) Representative: **Lunt, John Cooper et al**
**Monsanto Europe S.A. Patent Department**
**Avenue de Tervuren 270-272 Letter Box No 1**
**B-1150 Brussels (BE)**

**Description**

Background of the invention

This invention relates to antioxidants for rubber, of the type known generally as hydroquinolines, which antioxidants are usually prepared, in part, by the condensation with acetone of aniline or a substituted aniline.

Typical of the known materials of this type are oligomers of 1,2 - dihydro - 2,2,4 - trimethylquinoline, produced and sold under a number of tradenames. Such oligomers are usually prepared by first condensing acetone and aniline and then subjecting the condensate to polymerization conditions. Typically, the final product is a solid mixture of dimer, trimer and tetramer, together with small amounts of monomer and higher polymers.

Another similar known antioxidant material is 6 - dodecyl - 1,2 - dihydro - 2,2,4 - trimethylquinoline, which can be prepared by condensation of acetone with 4-dodecylaniline. The condensate is a liquid, and does not tend to polymerize.

Still another similar known hydroquinoline antioxidant material is 6 - ethoxy - 1,2 - dihydro - 2,2,4 - trimethylquinoline, which can be prepared by condensation of acetone with p-phenetidine (4-ethoxyaniline). This condensate is also a liquid, with little tendency to polymerize.

While the oligomerized product of the condensation of acetone and aniline is an effective and widely used product, its form has caused difficulties in use. Handling of solid ingredients in the preparation of rubber compounds can be a problem if the solid ingredients tend to dust, to cake, to pack, or to present other handling difficulties. In many instances, liquid forms of compounding ingredients are preferred, since liquids can be directly and automatically metered into an internal mixer, thus enabling precise control of amounts and avoiding the handling problems often associated with solid ingredients.

If, in the preparation of acetone-aniline condensates, the oligomerization step, above, is omitted, a liquid "monomeric" material can be obtained, but this material is usually too volatile to be an effective antioxidant.

Summary of the invention

It is an object of this invention to provide an improved rubber antioxidant of the hydroquinoline type, which antioxidant is in a stable liquid form at normal storage and handling temperatures, is not excessively volatile and provides antioxidant protection to the rubber equivalent to that obtained using solid, oligomeric 1,2 - dihydro - 2,2,4 - trimethylquinoline.

This object is obtained using an antioxidant which is the product of the condensation of acetone with a mixture comprising aniline and a $C_{3-15}$ alkylaniline and/or a $C_{1-12}$ alkoxyaniline.

The mixture of aniline and substituted aniline can contain from 1 to 99 mole percent of aniline, but at 99% aniline the product is relatively volatile unless it is polymerized, and the polymerized product is too viscous to be easily handled. At 1% aniline, the properties of the antioxidant are difficult to differentiate from those of the known alkyl-substituted or alkoxy-substituted hydroquinoline antioxidants.

Preferred is a material made from 25 to 75 mole percent aniline, the remainder being an alkylaniline or an alkoxyaniline as defined above, condensed with acetone. Materials within these limits exhibit good antioxidant activity and are fluid at room temperature.

Preferred alkylanilines are either single compounds, such as p-dodecylaniline, or mixtures of alkyl-substituted aniline compounds within the three-to-fifteen carbon range. The alkyl substituents can be straight chain or branched chain, and can be in the ortho, meta or para position. More preferred are the para-alkylanilines.·

Preferred alkoxyanilines are ortho-, meta-, or para-substituted lower alkoxy anilines. By "lower" is meant that the alkoxy groups have 1—3 carbon atoms. Especially preferred are para-alkoxy compounds, most especially p-phenetidine and p-anisidine.

The condensation reaction is performed at any suitable temperature, depending on the desired speed of the reaction, but is preferably performed at from 100° to 180°C. An appropriate catalyst can be employed to promote the condensation, and an acid, such as toluene sulfonic acid or HCl is most effective for this purpose.

In view of the fact that simple blends of the oligomeric 1,2 - dihydro - 2,2,4 - trimethylquinoline with 6 - dodecyl - 1,2 - dihydro - 2,2,4 - trimethylquinoline or 6 - ethoxy - 1,2 - dihydro - 2,2,4 - trimethylquinoline set up solid after a short time it was quite unexpected that the materials of the invention remained fluid and easy to handle.

Detailed description

Example I

To a three-neck round-bottom flask having a thermometer well and fitted with a distillation column are charged 32.7 g (0.125 mole) dodecylaniline, 11.6 g (0.125 mole) aniline and 2.4 g (0.025 mole, 10 mole %) concentrated hydrochloric acid. (The dodecyl aniline is actually a mixture of $C_{8-15}$ alkylated anilines which contains 45% $C_{12}$, 15% $C_{11}$ and 12% $C_{13}$ anilines). This mixture is heated to 140°C, and acetone is introduced below the liquid surface at the rate of about 10 ml/min. The temperature is maintained at 140°±2 and addition is continued for 12 hours. At the end of this period, heating is continued for 15 minutes to drive off

2

any remaining acetone. The contents of the flask are diluted with about 100 ml toluene, then washed with dilute NaHCO$_3$ until the pH of the aqueous layer is 8.5. The organic layer is then separated and dried (with Na$_2$SO$_4$), and concentrated by driving off the toluene. A viscous brown oil is recovered. G. C. analysis of the oil indicates 58% 6 - dodecyl - 1,2 - dihydro - 2,2,4 - trimethylquinoline and 6% dodecyl aniline; the remainder is presumed to be monomeric or oligomeric 1,2 - dihydro - 2,2,4 - trimethylquinoline.

Example II

The product of Example I is tested for its activity as an antioxidant in rubber, using a truck tire carcass stock based on the following masterbatch:

| Material | Parts by weight |
|---|---|
| Natural rubber—SMR 5 CV | 80.0 |
| Polybutadiene—Cis-4 BR 1203 | 20.0 |
| Carbon black—N550 | 50.0 |
| Processing oil—Sundex® 790 | 12.0 |
| Zinc oxide | 3.0 |
| Stearic acid | 2.0 |
| Resin—Piccopale 100® SF | 5.0 |
| Total | 172.0 |

This masterbatch is then mill-mixed, adding sulfur, accelerator (N - 2 - oxydiethylene - 2 - benzothiazylsulfenamide) and antioxidant as shown in Table I. Compared are commercial antioxidants Flectol® H (polymerized 1,2 - dihydro - 2,2,4 - trimethylquinoline) and Santoflex® DD (6 - dodecyl - 1,2 - dihydro - 2,2,4 - trimethylquinoline). The stocks, containing varying types and amounts of antioxidants are tested for physical properties, as shown in Table I. The tensile and elongation measurements are made in the conventional manner. Fatigue-to-failure tests are made using the Monsanto Fatigue-to-Failure Tester, with dumbbell samples flexed at 100 cycles per minute at an extension of 90%.

The physical test data in Table I show that the antioxidant of the invention has activity comparable to the known antioxidants.

Example III

To investigate the properties of a simple blend of dihydroquinoline antioxidants, two mixtures are made. Blend A contains equal parts by weight of polymerized 1,2 - dihydro - 2,2,4 - trimethylquinoline and 6 - dodecyl - 1,2 - dihydro - 2,2,4 - trimethylquinoline. Blend B contains two parts by weight of the former per 1 part of the latter. Each blend is physically combined, then heated to 160°—170°C with stirring, to form a homogeneous liquid mixture. The blends are cooled to room temperature, and are found to have solidified after a week.

TABLE I

| Stock | A | B | C | D | E | F | G | H |
|---|---|---|---|---|---|---|---|---|
| Masterbatch | 172.0 ———————————————————→ | | | | | | | |
| Insoluble sulfur 60 | 2.2 ————————————————————→ | | | | | | | |
| Accelerator | 1.2 ————————————————————→ | | | | | | | |
| Flectol® H | — | 1.5 | — | — | 3.0 | — | — | 1.5 |
| Santoflex® DD | — | — | 1.5 | — | — | 3.0 | — | 1.5 |
| Antioxidant of Example I | — | — | — | 1.5 | — | — | 3.0 | — |
| Physical properties Aged 72 hours @ 100°C % Retention of tensile | 58 | 78 | 77 | 69 | 81 | 78 | 79 | 81 |
| % Retention of elongation | 49 | 63 | 63 | 57 | 66 | 68 | 64 | 70 |
| Aged 15 days @ 70°C % Retention of tensile | 91 | 95 | 98 | 99 | 95 | 97 | 95 | 100 |
| % Retention of elongation | 82 | 83 | 83 | 89 | 81 | 84 | 82 | 90 |
| Fatigue-to-failure, kilocycles to failure Unaged | 71 | 92 | 86 | 84 | 93 | 105 | 98 | 99 |
| Aged 24 hrs. @ 100°C | 41 | 55 | 61 | 57 | 70 | 64 | 71 | 81 |

Example IV

In a manner similar to that of Example I, equimolar amounts of aniline and *p*-phenetidine are condensed with acetone. The resultant dark brown oily liquid remains fluid on standing. G. C. analysis gave the following:

33.2% Oligomeric 1,2-dihydro-2,2,4-trimethylquinoline
33.6% 6-Ethoxy-1,2-dihydro-2,2,4-trimethylquinoline
11.8% *p*-Phenetidine
 8.7% Monomeric 1,2-dihydro-2,2,4-trimethylquinoline.

Although the invention has been illustrated by typical examples, it is not limited thereto.

**Claims**

1. The product of the condensation of acetone with a mixture comprising aniline and
A) a $C_3$—$_{15}$ alkylaniline or
B) a $C_1$—$_{12}$ alkoxyaniline.
2. The product of Claim 1 wherein the mixture contains from 25 to 75 mole percent aniline.
3. The product of Claim 2 wherein the mixture is aniline and dodecylaniline.
4. The product of Claim 2 wherein the mixture is aniline and phenetidine.
5. The product of Claim 1 wherein the condensation is performed in the presence of an acidic catalyst at a temperature of from 100° to 180°C.
6. The product of Claim 5 which is further heated to form oligomers.
7. The product of Claim 5 wherein the catalyst is hydrochloric acid or toluenesulfonic acid.
8. The product of Claim 1 wherein the mixture contains both a $C_3$—$_{15}$ alkylaniline and a $C_3$—$_{12}$ alkoxyaniline.
9. The product of the condensation, in the presence of HCl at a temperature of from 100° to 180°C, of acetone with a mixture of aniline and a $C_3$—$_{15}$ alkylaniline, wherein the mixture contains from 25 to 75 mole percent aniline.

**Patentansprüche**

1. Kondensationsprodukt aus Aceton mit einer Mischung, die Anilin und
A) ein $C_3-_{15}$-Alkylanilin oder
B) ein $C_1-_{12}$-Alkoxyanilin enthält.
2. Kondensationsprodukt nach Anspruch 1, dadurch gekennzeichnet, daß die Mischung 25 bis 75 Mol-% Anilin enthält.
3. Kondensationsprodukt nach Anspruch 2, dadurch gekennzeichnet, daß die Mischung Anilin und Dodecylanilin umfaßt.
4. Kondensationsprodukt nach Anspruch 2, dadurch gekennzeichnet, daß die Mischung Anilin und Phenetidin umfaßt.
5. Kondensationsprodukt nach Anspruch 1, dadurch gekennzeichnet, daß die Kondensation in Gegenwart eines Säurekatalysators bei einer Temperatur von 100 bis 180°C durchgeführt wird.
6. Kondensationsprodukt nach Anspruch 5, dadurch gekennzeichnet, daß unter Bildung von Oligomeren weiter erhitzt wird.
7. Kondensationsprodukt nach Anspruch 5, dadurch gekennzeichnet, daß der Katalysator Salzsäure oder Toluolsulfonsäure darstellt.
8. Kondensationsprodukt nach Anspruch 1, dadurch gekennzeichnet, daß die Mischung sowohl ein $C_3-_{15}$-Alkylanilin als auch ein $C_1-_{12}$-Alkoxyanilin enthält.
9. Kondensationsprodukt, hergestellt in Gegenwart von HCl bei einer Temperatur von 100 bis 180°C aus Aceton mit einer Mischung aus Anilin und eines $C_3-_{15}$-Alkylanilins, dadurch gekennzeichnet, daß die Mischung 25 bis 75 Mol-% Anilin enthält.

**Revendications**

1. Produit de la condensation de l'acétone avec un mélange comprenant de l'aniline et
A) une (alkyle en $C_3$ à $C_{15}$) aniline ou
B) une (alcoxy en $C_1$ à $C_{12}$) aniline.
2. Produit selon la revendication 1, dans lequel le mélange contient de 25 à 75 moles % d'aniline.
3. Produit selon la revendication 2, dans lequel le mélange est de l'aniline et de la dodécylaniline.
4. Produit selon la revendication 2, dans lequel le mélange est de l'aniline et de la phénétidine.
5. Produit selon la revendication 1, dans lequel la condensation est effectuée en présence d'un catalyseur acide à une température de 100 à 180°C.
6. Produit selon la revendication 5, qui subit un chauffage supplémentaire pour former des oligomères.
7. Produit selon la revendication 5, dans lequel le catalyseur est l'acide chlorhydrique ou l'acide toluènesulfonique.
8. Produit selon la revendication 1, dans lequel le mélange contient à la fois une (alkyle en $C_3$ à $C_{15}$) aniline et une (alcoxy en $C_1$ à $C_{12}$) aniline.
9. Produit de la condensation, en présence de HCl à une température de 100° à 180°C, de l'acétone avec un mélange d'aniline et d'une (alkyle en $C_3$ à $C_{15}$) aniline, dans lequel le mélange contient de 25 à 75 moles % d'aniline.